# EUROPEAN PATENT APPLICATION

(11) **EP 4 834 964 A1**
(43) Date of publication of application: **04.11.2026**
(21) Application number: 24912674.9
(22) Date of filing: 20.12.2024
(51) Int. Cl.: C08F 290/00, A61K 8/893, A61Q 1/12, A61Q 17/04, C08F 299/08, C08G 77/42, C08G 77/48, C08L 83/05, C08L 83/07

(54) **BIODEGRADABLE SILICONE ELASTOMER PARTICLES HAVING HYDROPHILIC GROUP IN CROSSLINKED PART, AND COSMETIC COMPOSITION AND OTHER USES**

(30) Priority: 26.12.2023 JP 2023219852
(71) Applicant: Dow Toray Co., Ltd., Tokyo 140-8617 (JP)
(72) Inventor: SUGIURA Tsunehito, Ichihara-shi Chiba 299-0108 (JP); TANIGUCHI Hiroko, Ichihara-shi Chiba 299-0108 (JP); TAN Liyi, Ichihara-shi Chiba 299-0108 (JP); KANZAKI Yasue, Ichihara-shi Chiba 299-0108 (JP); SETOGUCHI Hiro, Ichihara-shi Chiba 299-0108 (JP); NAGAYAMA Kei, Ichihara-shi Chiba 299-0108 (JP)
(74) Representative: Elkington and Fife LLP
(86) International application number: PCT/JP2024/045219
(87) International publication number: WO 2025/142785

(57) **Abstract**

PROBLEM

To provide novel silicone elastomer particles that are highly biodegradable and can impart excellent feel and usability to cosmetic materials, and applications thereof.

SOLUTION

Biodegradable silicone elastomer particles having a structure in which at least two silicon atoms in the silicone elastomer particles are crosslinked by a divalent organic group containing a hydrophilic group, and the silicone elastomer particles contain a polyorganosiloxane structure expressed by

-(R₂SiO)ₘ-

(where R represents an unsubstituted alkyl group having 1 to 20 carbon atoms or an alkyl group having 1 to 20 carbon atoms substituted with a halogen atom, an aryl group having 6 to 22 carbon atoms, or a hydroxyl group, and m is a number ranging from 1 to 1000); as well as uses thereof.

## Description

### TECHNICAL FIELD

The present invention relates to novel silicone elastomer particles having a structure in which silicon atoms are crosslinked by divalent organic groups containing hydrophilic groups, which are highly biodegradable and capable of imparting excellent feel and texture to cosmetic materials. The biodegradable silicone elastomer particles have a crosslinked structure having a hydrophilic group that is active with respect to biodegradability. Therefore, primary particles thereof are expected to have a property of disintegrating with the generation of non-crosslinked siloxane molecules due to a decomposition reaction of microorganisms and the like in the natural world, and thus are expected to behave as biodegradable particles. Furthermore, the present invention relates to cosmetic raw materials, cosmetic compositions, organic resin additives and other applications including these biodegradable silicone elastomer particles.

### BACKGROUND ART

Silicone elastomer particles are cured from an addition reaction-curable or condensation reaction-curable silicone composition, and the particle diameter and oil absorbency thereof vary depending on the manufacturing method, but are widely used as stress-relieving agents or the like for cosmetic raw materials and thermoplastic resins. For example, as silicone particles having superior dispersibility, high lipophilicity, and superior storage stability, the present applicant has proposed silicone particles containing an alkylene group having 4 to 20 carbon atoms, which is obtained by curing a crosslinkable composition for forming silicone particles having a low amount of silicon atom-bonded hydrogen atoms per unit mass and containing an alkenyl group having 4 to 20 carbon atoms, such as a hexenyl group or the like, as described in Patent Document 1.

In addition, the applicant has focused on essential issues in conventional silicone elastomer particles. In other words, conventional silicone elastomer particles are formed through a crosslinking reaction of organopolysiloxane raw materials by hydrosilylation reactions or the like. The crosslinked structure is chemically stable, and even if these silicone elastomer particles were to be released into nature, it is undeniable that they would remain in nature without decomposing, at least for a short period of time, just like so-called microplastics. Therefore, there seems to be a latent desire in the market for silicone elastomer particles that perform well enough to smoothly replace or substitute existing silicone elastomer particles and that are expected to be highly biodegradable, in order to reduce risk to the global environment. In light of these potential market demands, the present applicants have proposed copolymer particles as described in Patent Documents 2 to 7. Furthermore, patent documents 8 and 9 propose silicone-based particles that are expected to be biodegradable.

On the other hand, Patent Documents 10 and 11 propose forming silicone elastomer particles using a crosslinking reaction between a polyoxyalkylene-containing compound and an organopolysiloxane raw material by a hydrosilylation reaction, or the like. However, such polyoxyalkylene crosslinked silicone elastomer particles are prone to swelling or deformation as a whole, so sufficient rubber properties are difficult to impart. As a result, individual elastomer particles cannot easily be provided with the same hardness and elasticity (rubber properties) as conventional products, and the feel of use and sensation derived from conventional products cannot easily be provided. Furthermore, since these silicone elastomer particles do not have crosslinking sites that are biodegradably active, even if the particles have hydrophilic sites within each molecule, the silicone elastomer particles cannot be expected to have high biodegradability, and there are no structures that would suggest biodegradability to a person of ordinary skill in the art.

On the other hand, Non-Patent Documents 1 to 3 disclose the biodegradability of polycarbonate compounds having a polyol terminal structure. Non-Patent Document 1 indicates that polycarbonates having aromatic groups, such as phenyl groups and the like, are difficult to biodegrade, whereas polycarbonates composed of aliphatic groups made of linear hydrocarbons are readily biodegradable. Non-Patent Documents 2 and 3 also describe the biodegradability of polycarbonates composed of aliphatic groups. There is no description nor suggestion of a radical polymerizable polycarbonate modified silicone compound having a plurality of polycarbonate structures with a relatively low degree of polymerization in the molecule and also having a polysiloxane structure with a specific structure, nor of silicone-polycarbonate copolymer particles obtained by radical polymerization thereof. In particular, there is no specific description nor suggestion regarding biodegradable silicone elastomer particles in which crosslinked structures between silicon atoms are formed by a crosslinking agent having a specific hydrophilic group.

Furthermore, Non-patent Document 4 discloses polydimethylsiloxane (PDMS) particles having a polycaprolactone (PCL) structure, which are described as being biodegradable and rubbery elastic particles with an elastic modulus. However, this document does not provide any specific description or suggestion regarding biodegradable silicone elastomer particles in which a crosslinked structure between silicon atoms is formed by a crosslinking agent having a specific hydrophilic group.

### RELATED ART DOCUMENTS

### Patent Documents

Patent Document 1: WO2017/191798 A1
Patent Document 2: WO2022/138346 A1
Patent Document 3: WO2023/120689 A1
Patent Document 4: WO2023/120690 A1
Patent Document 5: PCT/JP 2023/46100 A
Patent Document 6: PCT/JP 2023/46101 A
Patent Document 7: PCT/JP 2023/46102 A
Patent Document 8: WO2022/019179 A1
Patent Document 9: WO2023/238840 A1
Patent Document 10: 2001-163732 A
Patent Document 11: CN 111393679 A

### Non-Patent Documents

Non-Patent Document 1: Biodegradation of Aliphatic and Aromatic Polycarbonates (Trishul Artham, Mukesh Doble et al., Macro-Molecular Bioscience, 2007)
Non-Patent Document 2: Phylogenetic Affiliation of Soil Bacteria That Degrade Aliphatic Polyesters Available Commercially as Biodegradable Plastics (Tetsushi Suyama et al., Applied and Environmental Microbiology, 1998)
Non-Patent Document 3: Bacterial isolates degrading aliphatic polycarbonates (Tetsushi Suyama et al., FEMS Microbiology Letters, 1998)
Non-Patent Document 4: PCL-PDMS-PCL Copolymer-Based Microspheres Mediate Cardiovascular Differentiation from Embryonic Stem Cells (Liqing Song et al., TISSUE ENGINEERING: Part C Volume 23, Number 10, 2017)

### BRIEF DESCRIPTION OF THE DRAWINGS

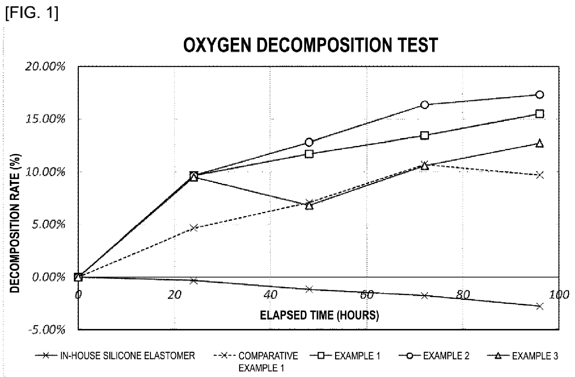

FIG.1 depicts the enzymatic degradation test results (test time - degradation rate %) of silicone elastomer particles in the examples and comparative examples.

### SUMMARY OF THE INVENTION

### PROBLEM TO BE SOLVED BY THE INVENTION

An object of the present invention is to solve the aforementioned problems, and provide biodegradable silicone elastomer particles having a crosslinked structure which is active with respect to biodegradability, and can provide texture and feel during use that is equal to or higher than conventional silicone elastomer particles when blended in a cosmetic composition or the like.

Furthermore, an object of the present invention is to provide a cosmetic raw material, an organic resin additive, and other applications with excellent feel during use, and the like by using the copolymer particles. Furthermore, an object of the present invention is to provide a cosmetic material composition containing the copolymer particles, with excellent feel during use, and the like.

Furthermore, an object of the present invention is to provide: copolymer particles that, in addition to performance equal to or better than conventional silicone elastomer particles, can be expected to be biodegradable, thereby reducing potential risks to the global environment, allowing for industrially sustainable and stable use, and making it possible to promote the material as biodegradable and eco-friendly to users and consumers in general who are concerned about global environmental impact, as well as to provide a synthesis raw material, and a use thereof.

### MEANS FOR SOLVING THE PROBLEM

In light of the foregoing and as a result of extensive research, the present inventors discovered that the aforementioned problem can be solved by biodegradable silicone elastomer particles having a structure in which at least two silicon atoms in the silicone elastomer particles are crosslinked by a divalent organic group containing a hydrophilic group, and the silicone elastomer particles contain a polyorganosiloxane structure expressed by

-(R₂SiO)ₘ-

(where R represents an unsubstituted alkyl group having 1 to 20 carbon atoms or an alkyl group having 1 to 20 carbon atoms substituted with a halogen atom, an aryl group having 6 to 22 carbon atoms, or a hydroxyl group, and m is a number ranging from 1 to 1000); as well as uses thereof.

Furthermore, the present inventors found that the above problem can be solved by cosmetic raw materials, organic resin additives, cosmetic materials, and organic resins containing these biodegradable silicone elastomer particles, and thereby achieved the present invention.

### EFFECT OF THE INVENTION

If the biodegradable silicone elastomer particles of the present invention are blended in a cosmetic material composition or the like, a texture and feel during use can be achieved which are equal to or higher than those of conventional silicone elastomer particles. Furthermore, the biodegradable silicone elastomer particles according to the present invention can be used to provide a cosmetic material raw material, an organic resin additive, and other applications containing the silicone elastomer particles. Furthermore, a cosmetic material composition containing the biodegradable silicone elastomer particles according to the present invention can provide a cosmetic material with excellent feel during use, and the like.

The biodegradable silicone elastomer particles of the present invention have a structure crosslinked by a polyorganosiloxane chain and a bivalent organic group having a hydrophillic group, and a divalent organic group having this hydrophillic structure is active in a biodegradable reaction, and is designed so that in a biodegradable environment, the crosslinked structure formed between silicon atoms in the copolymer particles is at least partially cleaved, and the primary particles are disintegrated in conjunction with the generation of polyorganosiloxane having a non-crosslinked structure. Therefore, the elastomer particles of the present invention provide biodegradability, which reduces the risk to the global environment, and appeal to users and consumers in general, who are concerned about global environment impact, as an eco-friendly material that can be used with a considerable sense of ease.

### PREFERRED MODE FOR CARRYING OUT THE INVENTION

In the present specification, the term "(meth)acryl" refers to "acrylic or methacrylic", and when expressed as "(meth)acryl modified" indicates that the modifying group may be one or both of an acryl modifying group and a methacryl modifying group. Similarly, the term "(meth) acryloxy" refers to "methacryloxy or acryloxy" and "(meth)acryloxy group-containing organic group" indicates either one or both of methacryloxy group-containing organic groups and acryloxy group-containing organic groups.

### Silicone elastomer particles

The biodegradable silicone elastomer particles of the present invention, applications thereof including cosmetic material raw materials in particular, a manufacturing method thereof, as well as a cosmetic material composition and an organic resin (including paint and coating agent) containing the same will be described in detail below.

### (A) Crosslinking agents having hydrophilic groups in each molecule

The silicone elastomer particles of the present invention have a structure crosslinked by divalent organic groups containing hydrophilic groups. Here, the divalent organic group containing a hydrophilic group is active in biodegradation reactions and imparts to the silicone elastomer particles a property that the crosslinked structure formed between silicon atoms in the copolymer particles will at least partially cleave in a biodegradable environment; and
(A) particularly preferably has a structure derived from at least one crosslinking agent selected from the following:
   (A1) a crosslinking agent having a hydrophilic group in each molecule where both terminal groups of each molecule are blocked with a hydrosilylation reactive silyl group expressed by R^{Alk}R₂SiO (where R^{Alk} is an organic group containing a carbon-carbon double bond, and R is an alkyl group having 1 to 20 carbon atoms that is unsubstituted or substituted with a halogen atom, an aryl group having 6 to 22 carbon atoms, or a hydroxyl group); and
   (A2) a crosslinking agent having at least two radical polymerizable functional groups in the molecule and containing a hydrophilic group in the molecule.
Note that when organohydrogenpolysiloxanes are crosslinked with polyether compounds or the like that simply have unsaturated bonds at the terminals, sufficient biodegradability may not be achieved, and in some cases, silicone elastomer particles with rubber properties may not be obtained.

The aforementioned crosslinked structure is preferably formed by a crosslinking reaction selected from radical polymerization reactions and hydrosilylation reactions, and preferably has a structure in which at least two silicon atoms in the silicone elastomer particles are crosslinked by one or more reactions selected from radical polymerization reactions and hydrosilylation reactions to silicon atom-bonded hydrogen atoms, preferably involving component (A). Note that component (A) may be reactive to both radical polymerization and hydrosilylation reactions to silicon atom-bonded hydrogen atoms. In such cases, component (A) may be introduced into the silicone elastomer particles by either or both of these reactions, but this is not particularly a limitation.

The crosslinking agent which is component (A) may be hydrosilylation reactive, and preferably is a crosslinking agent in which both terminals of the (A1) molecular chain are blocked with hydrosilylation reactive silyl groups expressed by R^{Alk}R₂SiO and which contains a hydrophilic group in each molecule. By using hydrophilic compounds having hydrosilylation reactive silyl groups at the terminals, the rubber properties and biodegradability of the resulting silicone elastomer particles can be further improved in some cases.

In the formula, R^{Alk} is an organic group containing a carbon-carbon double bond, which is a reactive group selected from (meth)acryl terminal groups expressed by -C(=O)-R¹-CR²=CH₂ and alkenyl terminal groups having 2 to 20 carbon atoms. Herein, R¹ is a chemical bond between CH and C(=O) or a divalent organic group with 1 to 20 carbon atoms, and is preferably a simple chemical bond as in "-C(=O)-CH=" or an alkylene group with 1 to 20 carbon atoms expressed by "-C(=O)-CmH₂ₘ-CH=" (m is a number in a range 0 to 20), such as CmH₂ₘ (note that m is 0 when R¹ is a chemical bond between CH and C(=O)). Furthermore, R² is a hydrogen atom or a methyl group, and provides an acryl-modified group or a methacryl-modified group or an alkenyl group.

R is an alkyl group with 1 to 20 carbon atoms unsubstituted or substituted by a halogen atom, an aryl group with 6 to 22 carbon atoms, or a hydroxyl group, and industrially, each R is preferably independently a methyl or phenyl group.

The crosslinking agent that is Component (A1) is a crosslinking agent that contains a hydrophilic group in each molecule, and the hydrophilic group has a polyether structure, particularly preferably a polyoxyethylene-polyoxypropylene block copolymer structure (hereinafter sometimes referred to as "Pluronic(R) structure"). The crosslinking agent (A1) incorporating these structures may also have a polyester structure or a polycaprolactone structure.

Examples of Component (A1) include modified polycaprolactone compounds having a polyoxyethylene-polyoxypropylene block copolymer structure within each molecule, as expressed by 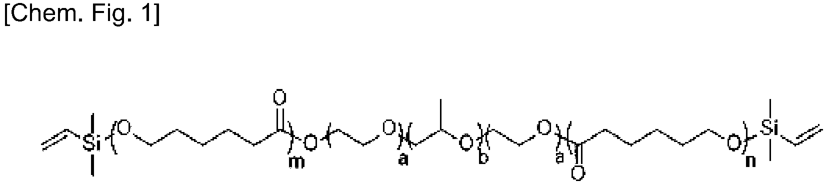 Here, m and n are positive numbers, and m + n is preferably in a range of 2 to 100, preferably 2 to 10, and more preferably 2 to 8. Furthermore, a represents the number of ethyleneoxy (EO) units, b represents the number of propyleneoxy (PO) units, a and b are positive numbers, and a + b is preferably in a range of 2 to 100, preferably 4 to 50, and more preferably 6 to 30. Note that in the formula, either one or both of the vinyl groups bonded to the silicon atom at the molecular chain terminal may be replaced by an organic group containing a carbon-carbon double bond other than the vinyl group corresponding to R^{Alk} described above.

The crosslinking agent which is component (A) may be radical polymerizable, and may be (A2) a crosslinking agent having at least two radical polymerizable functional groups in each molecule and containing a hydrophilic group in the molecule. Such component (A2) contains a hydroxyl (OH) group or a polyether structure that provides hydrophilicity to the crosslinked moiety, and preferably has two or more hydroxyl (OH) groups or a highly hydrophilic polyether structure. More specifically, component (A2) may be and preferably is one or more type of crosslinking agents selected from (a2-1) a sorbitan fatty acid ester or polyoxyalkylene sorbitan fatty acid ester having at least two radical polymerizable functional groups in each molecule, and (a2-2) a crosslinking agent containing at least two radical polymerizable functional groups and a polyether structure in each molecule.

The radical polymerizable functional group is not particularly limited, but similar to R^{Alk}, preferably is an organic group containing a carbon-carbon double bond, and is a reactive group selected from (meth)acrylic terminal groups expressed by -C(=O)-R¹-CR²=CH₂ and alkenyl terminal groups having 2 to 20 carbon atoms (R¹ and R² in the formula are the same groups as described above).

Component (A2) which is a sorbitan fatty acid ester or polyoxyalkylene sorbitan fatty acid ester, has at least two radical polymerizable functional groups in each molecule, and is preferably a crosslinking agent selected from sorbitan lauryl esters, sorbitan stearyl esters, sorbitan oleyl esters, and polyoxyethylene sorbitan oleyl esters. As an example, the following sorbitan fatty acid esters have two (meth)acrylic terminal groups in each molecule. In the formula, R' may be an alkyl group having 8 to 22 carbon atoms (such as a lauryl group, stearyl group, or oleyl group). Such sorbitan fatty acid esters or polyoxyalkylene sorbitan fatty acid esters are hydrophilic due to having a plurality of hydroxyl groups (OH) in each molecule, and these esters function as crosslinking agents in the present invention to improve the biodegradability of the silicone elastomer particles obtained.

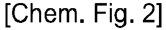

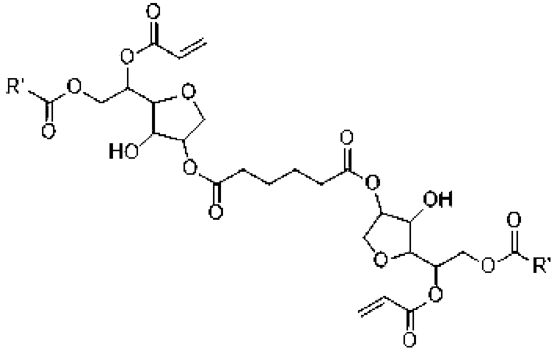

The crosslinking agent, which is component (A2) and contains at least two radical polymerizable functional groups and a polyether structure in each molecule, is preferably a crosslinking agent having a polyester structure or a polycarbonate structure, and may also have a polycaprolactone structure. The polyether structure in the crosslinking agent that is component (A2) is particularly preferably a polyoxyethylene-polyoxypropylene block copolymer structure ("Pluronic^{®} structure"). If the polyether structure in component (A2) is composed of ethyleneoxy (EO) units and propyleneoxy (PO) units, the structure is expressed as (EO)a(PO)b, where a and b are positive numbers, and a + b is preferably in a range of 2 to 100, preferably 4 to 80, and more preferably 6 to 70.

### Crosslinking Reactive Silicone Composition

The silicone elastomer particles of the present invention include at least: component (A) that is a crosslinking agent containing a hydrophilic group in each molecule as described above;
(B) at least one type of reactive organopolysiloxane selected from the following component (b1) and component (b2);
(b1) an organohydrogenpolysiloxane having at least three silicon atom-bonded hydrogen atoms in a molecule, and
(b2) an organopolysiloxane having at least three organic groups containing at least one type of (meth)acryloxy group selected from organic groups containing a methacryloxy group and organic groups containing an acryloxy group in each molecule; and
(C) one or more type of curing agent selected from (c1) a hydrosilylation reaction catalyst, and (c2) a radical polymerization initiator; the silicone elastomer particles are preferably silicone elastomer particles obtained by performing in water a crosslinking reaction of crosslinkable silicone emulsified particles obtained by emulsifying in water a crosslinkable silicone composition crosslinkable by one or more reactions selected from radical polymerization reactions and hydrosilylation reactions with a silicon atom-bonded hydrogen atom.

### Radical polymerization reactive silicone elastomer particles

The radical polymerization reactive silicone elastomer particles are obtained by radical polymerization of an organopolysiloxane having three or more silicon atom-bonded radical reactive functional groups such as a (meth)acryloxy group-containing organic group bonded to a silicon atom in each molecule and the aforementioned component (A2) in the presence of a radical polymerization initiator, and a crosslinked structure formed by a radical polymerization reaction of component (A) and a radical reactive functional group bonded to a silicon atom are provided between at least 2 silicon atoms in the silicone elastomer particles.

### Hydrosilylation-reactive silicone elastomer particles

The silicone elastomer particle is obtained by subjecting an organopolysiloxane (= organohydrogenpolysiloxane) having three or more silicon atom-bonded hydrogen atoms in each molecule and compound (A1) to a hydrosilylation reaction in the presence of a hydrosilylation reaction catalyst, and a crosslinked structure formed by a hydrosilylation reaction (addition reaction) between a hydrosilylation reactive silyl group in component (A1) and a silicon atom-bonded hydrogen atom is formed between at least two silicon atoms in the silicone elastomer particles.

The silicone elastomer particles of the present invention also contain a polyorganosiloxane structure (linear polysiloxane structure) expressed by:

-(R₂SiO)ₘ-

(where R represents an unsubstituted alkyl group having 1 to 20 carbon atoms, an alkyl group having 1 to 20 carbon atoms substituted with a halogen atom, an aryl group having 6 to 22 carbon atoms, or a hydroxyl group, and m is a number ranging from 1 to 1000).
Thereby, appropriate hardness and flexibility is provided to the silicone elastomer particles.

Industrially, R preferably independently represents a methyl group or phenyl group, and m is more preferably a number ranging from 10 to 800, and more preferably 20 to 750.

### Component (B)

Component (B) is a component that introduces the above polyorganosiloxane structure into the silicone elastomer particles by crosslinking with component (A) through one or more reactions selected from radical polymerization reactions and hydrosilylation reactions to silicon atom-bonded hydrogen atoms.

Component (b1) is an organopolysiloxane component that is crosslinked by component (A) by a hydrosilation reaction, and is characterized by having at least three silicon atom-bonded hydrogen atoms in each molecule, and the bonding positions of the hydrogen atoms in the molecules are not particularly limited.

Examples of an organic group other than a hydrogen atom that is bonded to a silicon atom contained by component (b1), include an alkyl group such as a methyl group, an ethyl group, a propyl group, a butyl group, or and an octyl group, and a methyl group is preferable. Examples of the molecular structure of the organohydrogen polysiloxane of component (b1), include straight-chain, branched-chain, and branched-cyclic, or a combination of one or more thereof. The number of silicon atom-bonded hydrogen atoms in a molecule is the average value of all molecules.

In particular, when the component (b1) is a linear organopolysiloxane (organohydrogenpolysiloxane), the silicone elastomer particles according to the present invention, when placed in a biodegradable environment, have an advantage that when the silicone elastomer particles are broken down by cleavage of the crosslinked structure, they are more likely to break down into non-crosslinkable, linear organopolysiloxanes, thus reducing the environmental impact and environmental risk.

The viscosity of component (b1) at 25 °C is 1 to 1,000 mPa·s, preferably 5 to 500 mPa·s. This is because if the viscosity of component (b1) at 25 °C is less than 1 mPa·s, component (b1) is easily volatilized from the crosslinkable composition containing it, and if the viscosity exceeds 1000 mPa·s, the curing time of the crosslinkable composition containing such component (a2) may become longer or may cause curing failure. Such component (a2) is not particularly limited, and examples include, dimethylsiloxane-methylhydrogensiloxane copolymer blocked at both molecular terminals with trimethylsiloxy groups, dimethylsiloxane-methylhydrogensiloxane copolymer blocked at both molecular terminals with dimethylhydrogensiloxy groups, dimethylpolysiloxane blocked at both molecular terminals with dimethylhydrogensiloxy groups, methylhydrogenpolysiloxane blocked at both molecular terminals with trimethylsiloxy groups, cyclic methylhydrogenpolysiloxane, and cyclic methylhydrogensiloxane-dimethylsiloxane copolymer.

Here, the value of H/Alk, which is the molar ratio (= reaction ratio in the hydrosilylation reaction) of the carbon-carbon double bond (Alk) in component (A1) to the silicon atom-bonded hydrogen atom amount (H) in component (b1) is preferably in a range of 0.7 to 1.2. The lower limit of said H/Alk is preferably 0.80 or more, 0.85 or more, 0.90 or more, 0.95 or more, and the upper limit is 1.15 or less, and preferably, 1.10 or less, or 1.05 or less. If the upper limit of H/Alk exceeds the aforementioned value, unreacted silicon atom-bonded hydrogen atoms tend to remain after the reaction; conversely, if the upper limit of H/Alk is less than the aforementioned value, unreacted component (B) and (meth)acryl terminal groups thereof tend to remain after the reaction. Since these are curing reactive groups, if a large amount remain in the particles, they may cause crosslinking reactions between particles over time, resulting in aggregation and poor dispersion of the resulting oil-containing silicone elastomer particles, and if reactive hydrogen atoms remain, they may cause the generation of flammable hydrogen gas over time. Particularly suitably, when the value of H/Alk is in the range of 0.9 to 1.1, particularly around 1.0, the curing reactive groups are completely consumed and the crosslinking reaction is terminated, and the aggregation over time between particles can be effectively suppressed.

Component (b2) must have at least three or more organic groups containing a (meth)acryloxy group on average in a molecule to form a crosslinked structure in a radical reaction with component (A). If only two or fewer organic groups containing a (meth)acryloxy group are present in a molecule on average, a sufficient crosslinked structure may not be formed, and practical silicone elastomer particles may not be obtained.

More specifically, the organic group containing a (meth)acryloxy group is a (meth)acryloxy group bonded to a silicon atom via a divalent organic group, and examples include 1 or more type of functional groups expressed by

-R²-O-C(=O)-C(R³)=CH₂

{where, R² is an alkylene group having 1 to 20 carbon atoms or a divalent linking group expressed by

(CH₂)ₚ-Si(CH₃)₂-O-Si(CH₃)₂-(CH₂)_{q}

(where p and q are numbers in a range of 1 to 20), and
R³ is either a hydrogen atom or a methyl group}.

The alkylene group, which is R² in the formula, may industrially be an alkylene group having 2 to 10 carbon atoms, and examples include propylene groups, butylene groups, hexylene groups, and the like. Furthermore, a divalent linking group expressed by (CH₂)ₚ-Si(CH₃)₂-O-Si(CH₃)₂-(CH₂)_{q} is a divalent linking group having a siloxane converter structure, and industrial examples include linking groups where p and q are each independently a number from 3 to 6.

Suitably, component (b2) is preferably a linear organopolysiloxane expressed by the following structural formula.

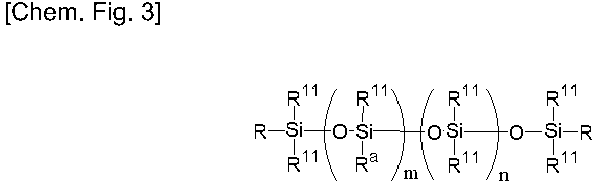

In Formula (1), each R¹¹ is independently an unsubstituted or halogen atom-substituted alkyl group (for example, a methyl group, or the like) having 1 to 20 carbon atoms, an aryl group (for example, a phenyl group, or the like) having 6 to 22 carbon atoms, or a hydroxyl group, and a methyl group or a phenyl group is preferable from an industrial point of view. R^{a} represents an organic group containing a (meth)acryloxy group, and is particularly preferably a (meth)acryloxy group bonded to a silicon atom by the aforementioned alkylene group or divalent linking group with a siloxane converter structure. R independently is a group represented by R¹¹ or R^{a}. m is a number of 1 or more, and n is a number of 1 or more. However, component (a) contains at least three organic groups containing a (meth)acryloxy group represented by R^{a} in a molecule, and therefore, when m = 1, R must both be R^{a}. In other words, the linear organopolysiloxane expressed by the structural formula above has an organic group containing a (meth)acryloxy group represented by R^{a} at one terminal site and a side chain site, a side chain site only, or both end sites and a side chain site of a siloxane molecule thereof, and preferably contains at least three organic groups containing a (meth)acryloxy group in a molecule.

m + n is the degree of siloxane polymerization of linear organopolysiloxane molecules excluding terminal siloxane structures. From the perspective of handling workability as a raw material and the ability to break down into fine linear siloxane molecules during emulsification biodegradation, m + n is preferably within a range of 10 to 800, more preferably 20 to 600, and particularly preferably 30 to 500. Furthermore, the viscosity of component (a) is particularly preferably a number between 20 and 10,000 mPa·s at 25°C.

Component (C) is a curing agent and is selected from (c1) a radical polymerization initiator and (c2) a hydrosilylation reaction catalyst, depending on the selection of component (A) and the reaction system.

Component (c1) is a radical initiator and is a component for promoting a radical polymerization reaction or radical copolymerization reaction of components (a1) and (B) described above. A conventionally known compound commonly used in radical polymerization methods is used as the radical initiator, and specific examples include: 2,2'-azobis(isobutyronitrile), 2,2'-azobis(2-methylbutyronitrile), 2,2'-azobis(2,4-dimethylvaleronitrile), and other azo-based compounds; benzoyl peroxide, lauroyl peroxide, tert-butylperoxy benzoate, tert-butylperoxy-2-ethylhexanoate, tert-hexylperoxy-2-ethylhexanoate, and other organic peroxides; and potassium persulfate, sodium persulfate, ammonium persulfate, and other persulfates. One type of these radical initiators may be used alone, or two or more types may be mixed and used together.

The amount of component (c1), which is a radical initiator, used is preferably within a range of 0.1 to 5 parts by mass relative to 100 parts by mass of the total of components (a1) and (B) above. In particular, when component (c1) is a water-soluble persulfate such as potassium persulfate or the like, component (c1) has an advantage of being extremely easy to add and react when crosslink reacting in water crosslinking reactive silicone emulsified particles, which are obtained by emulsifying a crosslinking reactive silicone composition in water via a radical polymerization reaction. Furthermore, upon completion of the radical polymerization reaction, it is particularly desirable to add aminomethylpropanediol or the like in a range of 0.1 to 5 parts by mass, for the purpose of stopping the reaction and neutralizing the solution by pH adjustment.

The timing of adding component (c1) to the crosslinkable composition can be selected according to the method of forming the silicone elastomer particles, and component (c1) can be added to the composition in advance, or component (a1) and component (B) can be supplied from different spray lines, and component (c1) can be added to either one, and then mixed during spraying. The silicone elastomer particles in the present invention are preferably formed via an aqueous suspension, which is formed via emulsification into water, and component (c1) may be added in advance to the crosslinking reactive silicone composition, or an emulsion containing component (c1) may be added separately to the water.

A chain transfer agent can be optionally added during the polymerization reaction of the crosslinking reactive silicone composition above. Specific examples of the chain transfer agent include mercapto compounds such as 2-mercaptoethanol, butyl mercaptan, n-dodecyl mercaptan, 3-mercaptopropyl trimethoxysilane, and polydimethylsiloxanes having a mercaptopropyl group; and halides such as methylene chloride, chloroform, carbon tetrachloride, butyl bromide, and 3-chloropropyl trimethoxysilane.

Component (c2) is a hydrosilylation reaction catalyst, which promotes the addition reaction (hydrosilylation reaction) between the carbon-carbon double bond in the alkenyl terminal group present in the above crosslinkable composition and a silicon atom-bonded hydrogen atom. Examples of preferred hydrosilylation reaction catalysts are hydrosilylation reaction catalysts containing platinum metal, such as platinic acid chloride, alcohol-modified platinic acid chloride, olefin complexes of platinic acid chloride, complexes of platinic acid chloride with ketones, complexes of platinic acid chloride with vinyl siloxane, platinum tetrachloride, platinum fine powder, platinum supported on an alumina or silica carrier, platinum black, olefin complexes of platinum, alkenylsiloxane complexes of platinum, carbonyl complexes of platinum, and thermoplastic organic resin powders such as methyl methacrylate resin, polycarbonate resin, polystyrene resin, and silicone resin containing these platinum-based catalysts. In particular, platinum alkenylsiloxane complexes such as a complex of platinum chloride with divinyltetramethyldisiloxane, a complex of platinum chloride with tetramethyltetravinylcyclotetrasiloxane, a platinum divinyltetramethyldisiloxane complex, and a platinum tetramethyltetravinylcyclotetrasiloxane complex can be preferably used. Note that as the catalyst for promoting the hydrosilylation reaction, a non-platinum-based metal catalyst such as iron, ruthenium, iron/cobalt, or the like may be used.

The amount of component (c2) added to the crosslinkable composition should be a catalytic quantity, and usually, an amount in which the amount of the platinum-based metal contained by component (c2) is in a range of 1 to 1000 ppm relative to the total mass of the crosslinkable composition described above is preferred, and an amount in a range of 5 to 500 ppm is even more preferred. Note that the amount of platinum metal in the silicone elastomer particles may be reduced by the method proposed by the present inventors in JP 2014-122316 A.

The timing of adding component (c2) to the crosslinkable composition can be selected according to the method of forming the silicone elastomer particles, and may be in the form of adding to the composition in advance, or in the form of supplying component (A) or component (B) from a different spray line, then adding component (c2) to one of the components, and then mixing during spraying. The oil-containing silicone elastomer particles of the present invention are preferably formed via an aqueous suspension, which is formed via emulsification into water, and component (c2) may be added in advance to the crosslinking reactive silicone composition, or an emulsion containing component (c2) may be added separately to the water.

The crosslinkable silicone composition described above may include a curing retarder represented by a hydrosilylation reaction inhibitor. Examples of such curing retarders are acetylene compounds, enyne compounds, organic nitrogen compounds, organic phosphorus compounds, and oxime compounds. Specific compounds include alkyne alcohols such as 2-methyl-3-butyn-2-ol, 3,5-dimethyl-1-hexyn-3-ol, 3-methyl-1-pentyn-3-ol, 2-phenyl-3-butyn-2-ol, and 1-ethynyl-1-cyclohexanol (ETCH); 3-methyl-3-trimethylsiloxy-1-butyn, 3-methyl-3-trimethylsiloxy-1-pentyn, 3,5-dimethyl-3-trimethylsiloxy-1-hexine, 3-methyl-3-penten-1-yne, and 3,5-dimethyl-3-hexene-1-yne enyne compounds; 1-ethynyl-1- trimethylsiloxycyclohexane, bis(2,2-dimethyl-3-butynoxy)dimethylsilane, methyl(tris(1,1-dimethyl-2-propynyloxy))silane, 1,3,5,7-tetramethyl-1,3,5,7-tetravinylcyclotetrasiloxane, and alkenylsiloxanes such as 1,3,5,7-tetramethyl-1,3,5,7-tetrahexenylcyclotetrasiloxane. The amount added is within the range of 0.001 to 5 mass parts per 100 mass parts of component (a), but can be designed as appropriate depending on the type of curing retarder used, the characteristics of the hydrosilylation reaction catalyst used, and the amount used.

The crosslinking reactive silicone composition above may contain one or more polymerization inhibitors from the perspective of preventing unintended side reactions and the like. For example, one or more types selected from hindered phenol-based polymerization inhibitors, hydroquinone-based polymerization inhibitors, and catechol-based polymerization inhibitors may be included. The amount used can be selected as appropriate, but the total concentration of the polymerization inhibitor is preferably 50 ppm by mass or less, and more preferably 30 ppm by mass or less, with respect to the sum of components (A) to (C) above.

The crosslinking reactive silicone composition may include a component other than the components above to the extent that the technical effects of the present invention are not impaired. For example, the composition may include: n-hexane, cyclohexane, n-heptane, or other aliphatic hydrocarbons; toluene, xylene, mesitylene, or other aromatic hydrocarbons; tetrahydrofuran, dipropyl ether, or other ethers; an organic solvent such as acetone, methyl ethyl ketone, methyl isobutyl ketone, or other ketones; a phenolic antioxidant, quinone antioxidant, amine antioxidant, phosphorus antioxidant, phosphite antioxidant, sulfur-based antioxidant, thioether antioxidant, or other antioxidant; a triazole light stabilizer, benzophenone light stabilizer, or other light stabilizer; a phosphate ester flame retardant, halogen flame retardant, phosphorus flame retardant, antimony flame retardant, or other flame retardant; one or more antistatic agents including cationic surfactants, anionic surfactants, and non-ionic surfactants; a dye; or a pigment.

The silicone elastomer particles of the present invention may optionally further have: (A) a structure in which part or all of a surface thereof is coated by one or more substance selected from organopolysiloxane resins, silica, and other silicone elastomer particles; (B) a mesoporous structure; (C) a structure containing an oil agent that is liquid at 40°C; and (D) a structure crosslinked by a silalkylene group having 2 to 20 carbon atoms, and a combination of arbitrary components providing these structures may be used.

### Hardness of silicone elastomer

Although the hardness of the silicone elastomer particles cannot be measured directly, the hardness can be measured indirectly by curing the crosslinkable silicone composition used to form the silicone elastomer particles serving as a raw material thereof. Specifically, the crosslinking reactive silicone composition is cured in a sheet form without emulsifying in water, and the hardness of the silicone elastomer sheet can be measured by a JIS A hardness tester as defined in JIS K6301. The hardness of the silicone elastomer according to the present invention varies depending on the type of the crosslinkable silicone composition, the amount of components (a)/(b) used, and the crosslink density, but is preferably within a range of 10 to 80. In addition, the preferred hardness is as described above.

### Formation of Silicone Elastomer Particles and Manufacturing Method Thereof

The silicone elastomer particles of the present invention can be provided by a method including a step of curing crosslinking reactive silicone emulsified particles, which are obtained by emulsifying in water with the crosslinking silicone composition used to form the silicone elastomer particles above, in the presence of (C) a curing agent to obtain spherical silicone elastomer particles.

The crosslinkable silicone composition used to form the silicone elastomer particles can be uniformly mixed using a mixer or other mechanical force.

In this method, silicone elastomer particles can be obtained by emulsifying and curing the aforementioned crosslinkable silicone composition in a surfactant aqueous solution. In addition, the particle diameter can be easily adjusted by adjusting the emulsion particle diameter. Examples of these surfactants include nonionic, anionic, cationic, betaine types, as well as water soluble polymers such as polyvinyl alcohols and the like. The particle diameter of the resulting silicone elastomer particles varies depending on the type and content of the surfactant. In order to prepare silicone elastomer particles having a small particle diameter, the amount of the surfactant added is preferably within a range of 0.5 to 50 parts by mass with respect to 100 parts by mass of the crosslinkable silicone composition.

An emulsifier is preferably used to uniformly disperse the crosslinking silicone composition in water in the form of crosslinking reactive silicone emulsified particles. Examples of the emulsifying machine include a homomixer, a paddle mixer, a Henschel mixer, a homo-disper, a colloid mill, a propeller agitator, a homogenizer, an in-line continuous emulsifier, an ultrasonic emulsifier, and a vacuum kneading machine.

The aqueous dispersion of the crosslinking reactive silicone emulsified particles prepared by the method above can then be heated or left at room temperature to cure the crosslinking reactive silicone emulsified particles in the aqueous dispersion to prepare an aqueous dispersion of silicone elastomer particles. When such an aqueous dispersion is heated, the heating temperature is preferably 100°C or lower from the perspective of hydrosilylation reactivity or radical polymerization reactivity, and is particularly preferably 10 to 95°C. Furthermore, examples of a method of heating the aqueous dispersion containing the crosslinking reactive silicone emulsified particles include a method of directly heating the water-based dispersion, and a method of adding the aqueous dispersion to hot water. The crosslinking reaction causes the liquid crosslinking reactive silicone emulsified particles to cure in water, forming an aqueous dispersion of silicone elastomer particles.

The resulting silicone elastomer particles of the present invention can be used as-is as an aqueous dispersion (aqueous suspension). In particular, the aqueous suspension form may be and is preferably used in cosmetic raw materials and the like. When added to a cosmetic material that uses an aqueous solution as a dispersion medium (such as hair cosmetic materials and the like), the silicone elastomer particles may be easily and uniformly dispersed to achieve a desired performance and feel during use by being added as an aqueous dispersion containing the silicone elastomer particles of the present invention.

Suitably, the silicone elastomer particles according to the present invention can be isolated by removing water from the aqueous dispersion of silicone elastomer particles. The method of removing water from the aqueous dispersion includes, for example, drying using a vacuum dryer, a hot air circulation oven, or a spray dryer. Note that the heating and drying temperature of the spray dryer must be set appropriately based on the heat resistance, crosslinking temperature, and the like of the silicone elastomer particles. Note that in order to prevent secondary aggregation of the resulting microparticles, the temperature of the silicone elastomer particles is preferably controlled to be equal to or lower than the glass transition temperature thereof. The silicone elastomer particles thus obtained can be recovered by a cyclone, a bag filter, or the like. Note that as a pretreatment for this operation, the dispersion may be concentrated by a method of heating and dehydration, filtration separation, centrifugation, decantation, or the like, and if necessary, the dispersion may be washed with water.

The silicone elastomer particles of the present invention may be surface treated, if necessary, to further improve the aggregation suppression effect of the silicone elastomer particles of the present invention. Furthermore, surface treatment with another known hydrophilic or hydrophobic treatment agent or the like may be applied. Optionally, the resulting silicone elastomer particles may be further coated with silica or other inorganic microparticles, silicone resin, or the like, in whole or in part on the surface thereof, as described above. Furthermore, the resulting silicone elastomer particles may be crushed or pulverized by a mechanical force if necessary, or classified using a known technique.

Furthermore, silicone elastomer particles obtained via such a manufacturing process may further improve the appearance, spreadability, and tactile sensation of the cosmetic material, particularly when used as a cosmetic raw material, and particles obtained via this manufacturing method tend to be more suitable for solving the problem of the present invention. Thus, one suitable form for achieving the technical effect of the present invention can be and is suitably defined by the manufacturing process.

The silicone elastomer particles according to the present invention are not particularly limited in terms of the average primary particle diameter thereof, but from the perspective of imparting a smooth texture and feel during use to the cosmetic material, not causing appearance defects and the like, storage stability and blending stability as a cosmetic material raw material, and the like, the average primary particle diameter measured by a laser diffraction scattering method is preferably within a range of 0.5 to 20 µm, more preferably within a range of 0.5 to 15 µm. Note that the particle diameter of the silicone elastomer particles can be controlled according to the crosslinking reactive silicone emulsified particles and crushing/classification process of the resulting silicone elastomer particles.

The shape of the silicone elastomer particles according to the present invention includes, for example, spherical, regular spherical shape, elliptical, and irregular shapes, and in particular, spherical and regular spherical shapes are preferred. Spherical silicone elastomer particles can be easily obtained by the method described later, in which the particles are prepared in the form of an aqueous suspension and dried using a vacuum dryer, hot air circulation oven, or spray dryer.

Furthermore, in the present invention, the crosslinking reactive silicone composition used to form the silicone elastomer particles is preferably within a range of 10 to 80 when cured in a sheet form, as measured by a JIS A hardness tester as defined in JIS K6301. If the JIS-A hardness of the rubber sheet measured by curing the crosslinking reactive silicone composition into a sheet form is within the range above, the resulting silicone elastomer particles will have sufficiently low aggregation and will tend to have exceptional flowability, dispersibility, smoothness, silkiness and a soft texture. Furthermore, by selecting the JIS-A hardness described above, it is possible to design or predict to some extent the feel during use, texture, and handling workability when added to a cosmetic material, and to improve stress relaxation properties when added to an organic resin. Note that when the silicone elastomer particles according to the present invention are used as a cosmetic raw material or stress relief agent or the like for organic resins, silicone elastomer particles having a JIS-A hardness in a range of 30 to 80, and particularly 50 to 80, described above are particularly preferably used.

Optionally, the silicone elastomer particles of the present invention may further contain a structure in which some or all of the surface thereof is covered with one or more types selected from organopolysiloxane resins, silica, and other silicone elastomer particles. Such coating may be expected to further reduce aggregation, control oil absorbency, improve texture, and the like.

Optionally, the silicone elastomer particles of the present invention may be mesoporous structures with micropores.

Optionally, the silicone elastomer particles of the present invention may contain an oil agent that is liquid at 40°C. The oil agent can be easily included in the silicone elastomer particles by emulsifying together in the crosslinking reactive silicone composition described later, and the inclusion of the oil agent may be expected to further reduce aggregation, control oil absorbency, improve texture, and the like.

### Cosmetic Raw Material and Cosmetic Material Composition

The silicone elastomer particles of the present invention are useful as cosmetic raw materials, and when blended in a cosmetic composition or the like, the particles are flexible and improve the feel and texture of the cosmetic materials, and the like, and provide outstanding handling workability, storage stability, and blending stability in systems as a cosmetic raw material.

In particular, the silicone elastomer particles of the present invention are superior to known silicone particles in terms of feel during use and texture, have a higher degree of freedom in formulation design, do not absorb oily ingredients over time when formulated in cosmetic materials, do not cause thickening or changes in texture, and when applied to the skin or hair, do not make the cosmetic material feel greasy or sticky. In addition, when the silicone elastomer particles of the present invention are used in combination with a UV protection component, the UV protection effect of the cosmetic material may be improved without impairing the texture and feel during use of the cosmetic material as compared with other powders or existing silicone elastomer particles.

Furthermore, the silicone elastomer particles of the present invention are active with respect to biodegradable reactions while providing performance that is equal or superior to conventionally known silicone elastomer particles. Moreover, a crosslinked structure formed between silicon atoms in the silicone elastomer particle is at least partially cleaved in a biodegradable environment, and primary particles of the silicone elastomer particles have a property of disintegrating with the generation of a polyorganosiloxane with a non-crosslinked structure. Therefore, the silicone elastomer particles are a low risk and low impact material for the global environment. Furthermore, use is possible as a replacement to conventionally known silicone elastomer particles, making it extremely versatile.

The cosmetic material composition containing the silicone elastomer particles of the present invention are not particularly limited in type, and examples include products such as: soaps, body shampoos, facial cleansing creams, and other cleaning cosmetic materials; toners, creams/milky lotions, packs, and other base cosmetic products; powders, foundation, and other base makeup cosmetic materials; lipstick, cheek rouge, eye shadow, eyeliners, mascara, and other facial cosmetic materials; nail polish and other makeup cosmetic materials; shampoos, hair rinses, hairdressing material, hair growth promoters, hair dye, and other hair cosmetic materials; perfume and eau de cologne, and other aromatic cosmetic materials; toothpastes; bath agents; and hair removal agents, shaving lotions, antiperspirants/deodorants, sunscreen agents, and other special cosmetic materials. Examples of the dosage forms of these cosmetic material compositions include aqueous liquid, oil-based liquid, emulsion, cream, foam, semi-solid, solid, and powder. These cosmetic material compositions can also be used by spraying.

In these cosmetic material compositions, the amount of the silicone elastomer particles described above is preferably within a range of 0.5 to 99.0 mass% in the cosmetic material composition, and particularly preferably within a range of 1.0 to 95 mass%. This is because if the amount of the silicone elastomer particles described above exceeds the upper limit of the range above, the effect as a cosmetic material is lost, and if the amount is below the lower limit of the range above, feel during use and the like of the cosmetic material composition and the like is less likely to be improved.

With respect to cosmetic material compositions (in particular, each formulation example) containing silicone particles (silicone rubber powder and the like) or silicone composite particles, which have been proposed in Patent Document 1 (JP H07-316014 A), Patent Document 2 (WO 2017/191798), and Patent Document 3 (JP H02-243612 A) as well as JP 2011-105663 A, JP 2011-168634 A, JP 2011-102354 A, and JP 2014-122316 A described above, the silicone elastomer particles of the present invention can be used in place of a part or all of these silicone-based particles, and there are cases where the feel during use as well as the production efficiency of the cosmetic material compositions proposed in these Patent Documents can be further improved. Note that it goes without saying that examples of cosmetic material compositions containing silicone particles (silicone rubber powder or the like) or silicone composite particles that can be blended with the silicone elastomer particles of the present invention are not limited to the above, and formulations may be designed in which some or all of silicone particle components in commercially available cosmetic materials are replaced by the silicone elastomer particles of the present invention, using a technique common to a person of ordinary skill in the art.

Furthermore, the silicone elastomer particles of the present invention can be applied to replace some or all of these silicone-based particles with respect to the applications and formulations of cosmetic material compositions disclosed in the aforementioned Patent Documents and the like, and the use therein is encompassed within the scope of the invention of the present application. As an example, the silicone elastomer particles of the present invention may be and are preferably used in combinations of arbitrary components, such as cosmetic product media (aqueous media or oil-based media), oil-based media (including oil agents and volatile oil agents), water, colorants, pigments, ultraviolet light blocking components, alcohols, water-soluble polymers, film-forming agents, oil agents, oil-soluble gelling agents, organically modified clay minerals, surfactants, resins, salts, moisturizers, preservatives, antimicrobial agents, antioxidants, pH adjusters, chelating agents, refreshing agents, anti-inflammatory agents, skin brightening agents (such as whitening agents, cell activators, skin roughness improvement agents, blood circulation promoters, skin astringents, and anti-seborrheic agents), vitamins, amino acids, nucleic acids, hormones, inclusion compounds, bioactive substances, medicament active components, perfumes, and the like, by selecting a method or quantitative range similar to those disclosed in Patent Document 2 (International Patent Publication WO2017/191798).

In particular, the silicone elastomer particles of the present invention have superior feel during use, texture, handling workability, storage stability, dispersibility, and high oil absorption properties that are equal or superior to conventionally known silicone particles, silicone composite particles coated with silsesquioxane, and silicone particles containing an oil agent. Therefore,
(1) Cosmetic material compositions and formulations containing oil-based media such as oil agents and the like (oil-based cosmetic material raw materials);
(2) Cosmetic material compositions and formulations containing lipophilic ultraviolet light blocking components (such as octyl paramethoxycinnamate and the like); and
(3) Cosmetic material compositions and formulations containing inorganic powders such as colorants and pigments,
can provide a particularly favorable appearance, feel of use, and the like. These specific formulations are further described in detail in the Examples.

In addition thereto, the silicone elastomer particles of the present invention can be easily designed for aqueous dispersions. Therefore, in aqueous cosmetic material compositions and formulations, the silicone elastomer particles provide excellent formulation design freedom and blending stability, and thus can achieve a suitable feel during use. These specific formulations are further described in detail in the Examples.

The cosmetic material of the present invention can be easily manufactured by simply uniformly mixing the cosmetic product raw material of the present invention and other cosmetic product raw materials as described above. As mixing means, various mixing and kneading devices normally used in the manufacture of cosmetic products can be used. Examples of such devices include a homomixer, a paddle mixer, a Henschel mixer, a homodisper, a colloidal mixer, a propeller agitator, a homogenizer, an in-line continuous emulsifier, an ultrasonic emulsifier, and a vacuum kneading machine.

### Organic Resin Additives and Organic Resins, Paints, and Coating Agents

The silicone elastomer particles of the present invention are also extremely useful as an organic resin additive due to the properties described above. Specifically, the silicone elastomer particles of the present invention have superior uniform dispersibility in organic resins and, if desired, excellent stress relief properties, and the like. In addition, the particles have very excellent handling workability and storage stability because aggregation is less likely to occur even after long-term storage. Furthermore, a member, paint film, or coating film obtained by curing an organic resin containing the silicone elastomer particles has improved flexibility (including the softness of a coating layer), durability, and adhesion and conformability to the substrate, is particularly pliable, and has superior thermal shock resistance. Therefore, it is extremely useful as a highly functional organic resin, paint, or coating agent for use in electronic materials.

### Organic Resin

A curable organic resin composition or a thermoplastic resin is suitably exemplified as an organic resin containing the silicone elastomer particles of the present invention. Of these, curable resins are suitable for electronic materials such as semiconductor substrates and the like. More specifically, examples of the curable organic resin composition include, phenolic resin, formaldehyde resin, xylene resin, xylene-formaldehyde resin, ketone-formaldehyde resin, furan resin, urea resin, imide resin, melamine resin, alkyd resin, unsaturated polyester resin, aniline resin, sulfone-amide resin, silicone resin, epoxy resin, and copolymer resins of these resins, and two or more of these curable resins can be combined. In particular, the curing resin is preferably at least one type selected from the group consisting of an epoxy resin, a phenolic resin, an imide resin, and a silicone resin. The epoxy resin can be any compound containing glycidyl or alicyclic epoxy groups, and examples include o-cresol novolac epoxy resins, phenol novolac epoxy resins, biphenyl epoxy resins, bisphenol A epoxy resins, bisphenol F epoxy resins, dicyclopentadiene epoxy resins, naphthalene epoxy resins, anthracene epoxy resins, naphthol aralkyl epoxy resins, polyvinylphenol epoxy resins, diphenylmethane epoxy resins, diphenylsulfone epoxy resins, triphenolalkane epoxy resins, cresol-naphthol co-condensation epoxy resins, bisphenylethylene epoxy resins, fluorene epoxy resins, stilbene epoxy resins, spiro-coumarone epoxy resins, norbornene epoxy resins, terpene epoxy resins, phenolcyclohexane epoxy resins, halogenated epoxy resins, imide-group-containing epoxy resins, maleimide-group-containing epoxy resins, allyl group-modified epoxy resins, and silicone-modified epoxy resins. Examples of this phenolic resin include a polyvinylphenol type, a phenol novolac type, a naphthol type, a terpene type, a phenol dicyclopentadiene type, a phenol aralkyl type, a naphthol aralkyl type, a triphenol alkane type, a dicyclopentadiene type, a cresol naphthol co-condensation type, and a xylene-naphthol co-condensation type. An example of a silicone resin is an epoxy-modified silicone resin generated by a reaction between an epoxy resin and a silanol group or a silicon atom-bonded alkoxy group in the silicone resin. Examples of the curing mechanisms of such curable resins are thermal curing, high energy beam curing such as ultraviolet light, radiation, and the like, moisture curing, condensation reaction curing, and addition reaction curing. The properties of such curable resins at 25 °C are not limited, and may be in either a liquid state or a solid state that softens upon heating.

Another optional component such as a curing agent, curing promoter, filler, photosensitizer, higher fatty acid metal salt, ester wax, plasticizer, or the like can be added to the organic resin containing the silicone elastomer particles of the present invention. Examples of curing agents include: organic acids such as carboxylic acids, sulfonic acids, and the like and anhydrides thereof; organic hydroxy compounds; organic silicon compounds having a silanol group, an alkoxy group, or a halogeno group; and primary or secondary amino compounds, and two or more types can be combined. Examples of the curing promoter include: tertiary amine compounds, and organic metal compounds such as aluminum, and zirconium; organophosphorus compounds such as phosphine; other heterocyclic amine compounds, boron complex compounds, organic ammonium salts, organic sulfonium salts, organic peroxides, and catalysts for hydrosilylation. Examples of these fillers include: fibrous fillers such as glass fiber, asbestos, alumina fiber, ceramic fiber composed of alumina and silica, boron fiber, zirconia fiber, silicon carbide fiber, metal fiber, polyester fiber, aramid fiber, nylon fiber, phenolic fiber, and natural animal and plant fiber; and powdered fillers such as fused silica, precipitated silica, fumed silica, calcined silica, zinc oxide, calcined clay, carbon black, glass beads, alumina, talc, calcium carbonate, clay, aluminum hydroxide, barium sulfate, titanium dioxide, aluminum nitride, silicon carbide, magnesium oxide, beryllium oxide, Kaolin, mica, and zirconia. Two or more of these can be combined. In the case of epoxy resins, it is particularly preferable to include an amine curing agent.

The silicone elastomer particles of the present invention may be added as an additive to a thermoplastic resin other than those described above, and may be used as a modifier of physical properties such as surface lubricants, stress relief agents, and the like, or modifiers of optical properties such as light scattering agents and the like. The type of thermoplastic resin is not particularly limited, and may be at least one polymer selected from a group consisting of: polycarbonate resins; polyester resins; polyether resins; polylactic acid resins; polyethylene, polypropylene, ethylene-propylene copolymers, and other polyolefin resins; polystyrene resins; styrene copolymers; tetrafluoroethylene and other fluorine-based polymers; polyvinyl ethers; and cellulose-based polymers, or a composite resin containing a combination of these. The silicone resin coated silicone elastomer particles of the present invention can be uniformly dispersed in these thermoplastic resins (including master batches) using a mixing device such as a biaxial or single-axis extruder, a kneader mixer, or the like, and may be molded into a desired shape, such as a film form or the like, for use.

The added amount of the silicone elastomer particles of the present invention may be selected as appropriate according to the physical properties required of the organic resin, but is generally within a range of 0.1 to 30 parts by mass with respect to 100 parts by mass of the organic resin, and may be within a range of 0.5 to 10 parts by mass. The reason is that if the amount of the particles added is less than the lower limit, the performance such as stress relief properties for the resin or the like may become insufficient, and the pliability and thermal shock resistance of the resulting cured organic resin product may decrease, and in particular, the thermal shock resistance tends to decrease after moisture absorption. On the other hand, if the amount exceeds the upper limit, the organic resin or the paint/coating agent after blending may become thickened and the handling workability may decrease, and the mechanical properties of the resulting organic resin cured product tend to decrease.

Furthermore, the silicone elastomer particles of the present invention are superior in stress relief when added to an organic resin, and thus may be added to an epoxy resin or the like for printed wiring boards to form a prepreg. Furthermore, a copper foil containing filler particles for a printed wiring board provided with a resin layer containing the silicone elastomer particles of the present invention on one side of the copper foil may be formed and used for a copper clad laminate (CCL) application.

### Paints, Coating Agents

Examples of paints and coating agents containing the silicone elastomer particles of the present invention include ambient temperature curing types, ambient temperature drying types, and heat curing types, with examples based on the properties thereof including aqueous types, oil-based types, and powdered types, and examples based on the vehicle resin including polyurethane resin paint, butyral resin paint, long oil phthalate resin paint, alkyd resin paint, amino alkyd resin paint made up of amino resin and alkyd resin, epoxy resin paint, acrylic resin paint, phenol resin paint, silicone modified epoxy resin paint, silicone modified polyester resin paint, and silicone resin paint.

The added amount of the silicone elastomer particles of the present invention can be selected as appropriate according to the physical properties required for the paint/coating agent, but in order to impart uniform and soft matting properties to the resulting coating film, the added amount is preferably within a range of 0.1 to 150 parts by mass with respect to 100 mass parts of solid content of the paint, more preferably within a range of 0.1 to 100 parts by mass, even more preferably 0.1 to 50 parts by mass, and particularly preferably 0.1 to 20 parts by mass. If the amount of the particles added is less than the lower limit, performance such as matting, adhesion, and stress relief properties of the coating film may be insufficient. If the amount of the particles exceeds the upper limit, the organic resin and the paint/coating agent after blending may become thickened and the handling workability may decrease.

The paints and coating agents containing the silicone elastomer particles of the present invention may contain: alcohols such as methanol, and ethanol; ketones such as methyl ethyl ketone, and methyl isobutyl ketone; esters such as ethyl acetate, butyl acetate, and cellosolve acetate; amides such as N,N-dimethylformamide; olefins such as hexane, heptane, and octane; organic solvents such as toluene, xylene, and other aromatic hydrocarbons; known inorganic fillers such as reinforcing silica; organic fillers; curing promoters; silane coupling agents; carbon black and other pigments; dyes; antioxidants; thickeners containing a high molecular weight compound; flame retardants; and weather resistance imparting agents.

### Eco-Friendly Material

As described above, unlike conventional non-biodegradable thermoplastic resin particles and silicone particle materials, the silicone elastomer particles of the present invention are expected to be biodegradable in a biodegradable environment, where crosslinked structures formed between silicon atoms within the silicone elastomer particles are at least partially cleaved, and primary particles of the silicone elastomer particles have a property of disintegrating with the generation of a polyorganosiloxane with a non-crosslinked structure. Therefore, use is possible as an "eco-friendly" cosmetic raw material with low environmental burden and environmental risk that complies with regulations on microplastics and the like, and appeal as a biodegradable and "eco-friendly" material is expected for users and consumers who are concerned about global environmental impact.

### EXAMPLES

The biodegradable silicone elastomer particles of the present invention and the manufacturing method thereof will be described in detail based on examples and comparative examples. However, the present invention is not limited only to these examples. The viscosity in the examples is the value at 25°C. The properties of each silicone particle were measured as follows. Note that in the examples and the like, unless otherwise specified, silicone particles is a generic term for particles made of a silicone cured product (cured silicone particles), and does not include emulsions.

### Average Primary Particle Diameter of Emulsion Particles

The emulsion before the addition of the radical polymerization initiator or before the addition of the hydrosilylation catalyst was measured using a laser diffraction particle diameter distribution analyzer (LS-230 from Beckman Coulter), and the median diameter (particle diameter corresponding to 50% of the cumulative distribution, 50% particle diameter) was used as the average particle diameter.

### Average Secondary Particle Diameter of Silicone Particles (Powder)

Using ethanol as a dispersion medium, the particle diameter of the cured silicone particles was measured with a laser diffraction particle diameter distribution analyzer (Mastersizer 3000 from Malvern Panalytical), and the median diameter of the cured silicone particles in ethanol (particle diameter corresponding to 50% of the cumulative distribution, D90, µm) and the arithmetic dispersion (particle diameter distribution SD, µm²) values were obtained. For the measurement sample, cured silicone particles (1 g) and ethanol (100 mL) were dispersed in a 300 mL cup using stirring blades and an ultrasonic vibrator.

### Synthesis Example 1: Alkenyl-modified polycaprolactone compound No. 1

A four-neck separable flask was charged with 35.12 parts by weight of Pluronic L-31 (trade name, manufactured by ADEKA Corporation, diol type polyoxyethylene-polyoxypropylene copolymer, molecular weight: about 1100), 49.70 parts by weight of chloroform, and 0.22 parts by weight of triazabicyclodecene. 14.58 parts by weight of ε-caprolactone was added dropwise with aeration of N₂. Stirring was performed at room temperature for 4 hours. After the reaction, 0.38 parts by weight of benzoic acid was added. After allowing to stand overnight, chloroform was removed by heating under reduced pressure with N₂ bubbling to obtain a transparent polymer. 83.21 parts by weight of the obtained polymer and 16.79 parts by weight of vinylsilazane were added. The mixture was heated to 50°C under N₂ bubbling, 0.02 parts by weight of trifluoromethanesulfonic acid was added, and the mixture was reacted for 4 hours. After filtration, the by-components were removed by N₂ bubbling to obtain a transparent polymer. Based on H-NMR and Si-NMR analysis, a peak derived from a vinylsiloxy group appeared in the polymer, so the polymer was polycaprolactone having a terminal modified with an vinylsiloxy group (alkanyl-modified EOPO polycaprolactone compound No. 1) having the following structure. 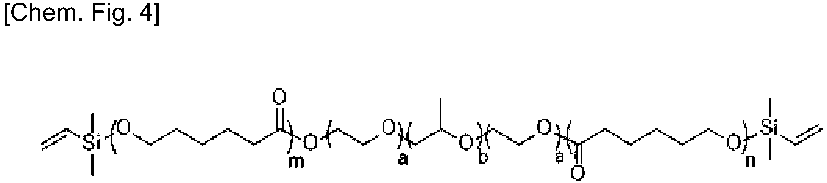 (where m + n = 4.0 and a + b = 18)

### Example 1: Silicone elastomer particle No. 1 (hydrosilylation reaction type)

A linear organohydrogen polysiloxane (viscosity 55 mm²/s) and alkenyl-modified polycaprolactone compound No. 1 were uniformly mixed at room temperature at a mass ratio of 15:85. Thereafter, this composition was dispersed in an aqueous solution at 25°C that was composed of 30 parts by mass of purified water and 0.5 parts by mass of polyoxyethylene alkyl (C12-14) ether. Afterwards the mixture was uniformly emulsified using a colloid mill, and 526 parts by mass of purified water was added for dilution to prepare the emulsion. Next, an isopropyl alcohol solution of platinum chloride (in an amount that allows platinum metal to be 10 ppm by mass in the composition) was added to the emulsion as an aqueous dispersion with polyoxyethylene alkyl (C12-14) ether and pure water, and the emulsion was stirred. Subsequently, the silicone rubber was let stand at 50 °C for 4 hours to prepare a uniform aqueous suspension of elastomer particles. The aqueous suspension was then filtered and the residue was dried in an oven at 50°C for five hours to obtain silicone elastomer particle No. 1. The average primary and secondary particle diameters of the resulting elastomer particles were 0.95 µm and 30.2 µm, respectively.

### Synthesis Example 2: Methacrylic-modified silicone polymer

91.03 parts by weight of octamethylcyclosiloxane and 0.01 parts by weight of MEHQ (hydroquinone monomethyl ether, polymerization inhibitor), and 6.54 parts by weight of 3-methacryloxypropylmethyldimethoxysilane were placed in a 4-neck separable flask. The mixture was heated while stirring at 200 rpm and blowing 2%O₂ N₂, and when the temperature reached 50°C, 0.05 parts by weight of trifluoromethanesulfonic acid and 1.52 parts by weight of water were added. After reacting at 55°C for 1 hour, the liquid temperature was heated to 70°C. Furthermore, the pressure was reduced to 100 mmHg to remove the methanol produced as a by-product for about 1 hour. Thereafter, 0.85 parts by weight of hexamethyldisiloxane and a trace amount of water was added, and the reaction was carried out for 3 hours. After the reaction, ammonia gas was bubbled to neutralize the trifluoromethanesulfonic acid, and the generated salt was removed by filtration using diatomaceous earth. The filtrate was subjected to reduced pressure processing at 150°C for 3 hours to remove volatile components. According to C, Si-NMR analysis, (a1-1) a methacryl-modified silicone polymer was obtained, having 214 units of dimethylsiloxane and 5 units of methacryl group-introduced siloxane, with a viscosity of 472 mPas.
This is a methacrylic-modified silicone polymer expressed by the formula 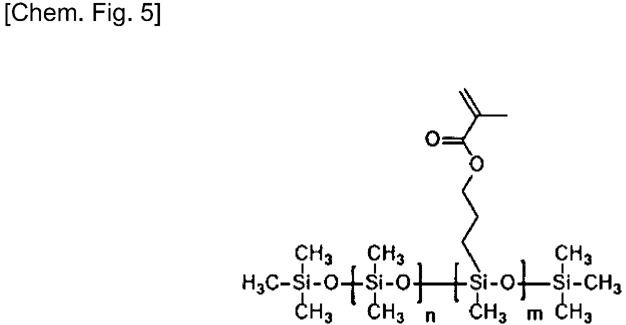 (where m = 5, n = 214) (viscosity at 25°C: 472 mPas).

### Synthesis Example 3: Acrylic-modified cross-linked sorbitan monolaurate

35.37 parts by weight of sorbitan monolaurate and 45.18 parts by weight of chloroform were placed in a four-necked separable flask. The mixture was stirred at room temperature while supplying nitrogen gas, and then 9.81 parts by weight of adipoyl chloride was added dropwise to maintain the exothermic temperature below 30°C. After reacting at room temperature for 5 hours, 9.63 parts by weight of potassium carbonate was added and the mixture was reacted for 1 hour. Excess potassium carbonate was removed by filtration. The resulting solution was heated to 70°C. Furthermore, the pressure was reduced to 100 mmHg to remove the chloroform produced as a by-product for about 1 hour. Subsequently, the pressure was reduced to 5 mmHg, and the chloroform was removed at 70°C for 2 hours. 33.94 parts by weight of the obtained cross-linked sorbitan monolaurate, 44.66 parts by weight of chloroform, 0.05 parts by weight of MEHQ (= hydroquinone monomethyl ether, polymerization inhibitor), and 10.63 parts by weight of potassium carbonate were placed in a four-necked separable flask. 10.71 parts by weight of acryloyl chloride was added dropwise at room temperature, while stirring and aerating with 2% O₂ in N₂, ensuring the temperature did not exceed 30°C, and the mixture was reacted at room temperature for 5 hours. Water was added until the solution became neutral, as measured by pH test paper. The solution was heated to 70°C, then the pressure was reduced to 100 mmHg, and the chloroform was removed over approximately one hour. Subsequently, the pressure was reduced to 5 mmHg, and the chloroform was removed at 70°C for 2 hours. H-NMR and C-NMR analysis revealed that the polymer had the following structure, namely a sorbitan lauryl ester crosslinked with adipoyl chloride and modifed with an acrylic group ((meth)acrylic-modified sorbitan lauryl compound). 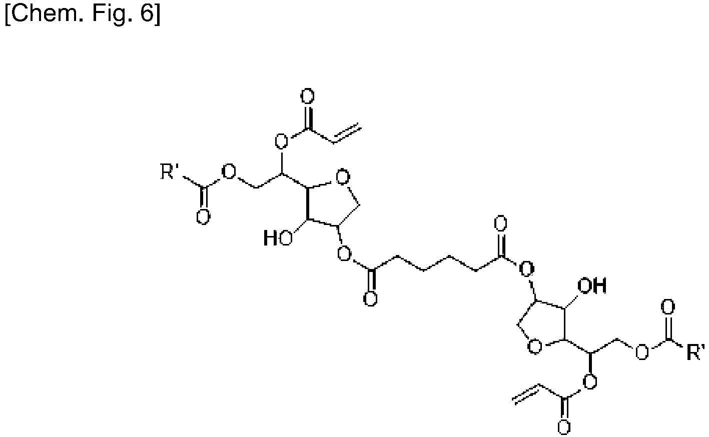 (where R' is a lauryl group)

### Example 2: Silicone elastomer particles No. 2 (radical polymerization type)

The aforementioned methacrylic-modified silicone polymer and (meth)acrylic-modified polysorbitan lauryl compound were uniformly mixed at room temperature in a mass ratio of 17.2:82.8. Next, this composition was dispersed in an aqueous solution at 25°C containing 0.27 parts by mass of GOHSENOL EG-05C and 0.53 parts by mass of GOHSENOL EG-18P in 46 parts by mass of pure water, and after uniform emulsification using a colloid mill, 526 parts by mass of pure water was added. After heating in a 1 L flask to 70°C, an aqueous solution of 0.5 g potassium persulfate (manufactured by Sigma-Aldrich) dissolved in 9.5 g water was added dropwise over one minute. The emulsion was stirred at 70°C for three hours and then at 80°C for two hours to perform radical polymerization to prepare a uniform aqueous suspension of silicone rubber particles. The aqueous suspension was then filtered and washed with 200 mL of ethanol and 100 mL of acetone. The residue was dried in an oven at 70°C for 5 hours to obtain silicone elastomer particles No. 3. The average primary and secondary particle diameters of the resulting silicone elastomer particles were 2.84 µm and 318 µm, respectively.

### Synthesis Example 4: Acrylic-Modified Polycaprolactone Compound

A four-neck separable flask was charged with 42.79 parts by weight of Pluronic L-64 (trade name, manufactured by ADEKA Corporation, diol type polyoxyethylene-polyoxypropylene copolymer, molecular weight: about 2900), 49.86 parts by weight of chloroform, and 0.10 parts by weight of triazabicyclodecene. 7.07 parts by weight of ε-caprolactone was added dropwise with aeration of N₂. Stirring was performed at room temperature for 4 hours. After the reaction, 0.18 parts by weight of benzoic acid was added. After allowing to stand overnight, chloroform was removed by heating under reduced pressure with N₂ bubbling to obtain a transparent polymer. 44.53 parts by weight of the obtained polymer, 0.02 parts by weight of MEHQ (hydroquinone monomethyl ether, polymerization inhibitor), and 3.62 parts by weight of potassium carbonate were placed in a four-necked separable flask and stirred at room temperature. 3.65 parts by weight of acryloyl chloride was added dropwise, ensuring the temperature did not exceed 30°C, and the mixture was allowed to react for 8 hours. After leaving overnight, the reaction was allowed to continue at room temperature for another 8 hours. A small amount of water was added, and then more water was added until the solution became neutral, as measured by pH test paper. A small amount of sodium sulfate was added, and the mixture was filtered. The resulting filtrate was heated to 70°C, the pressure was reduced to 100 mmHg, and chloroform was removed for about 1 hour. Subsequently, the pressure was reduced to 5 mmHg, and the chloroform was removed at 70°C for 2 hours. H-NMR and C-NMR analysis revealed that the polymer was a polycaprolactone modified with acrylic groups at the terminals ((meth)acrylic-modified polycaprolactone compound). 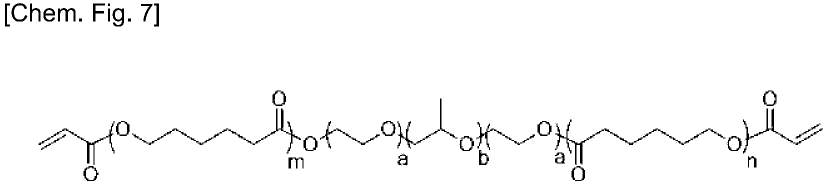 (where, m + n = 4.0, a + b = 43 to 45)

### Example 3: Silicone elastomer particles No. 3 (radical polymerization type)

The aforementioned methacrylic-modified silicone polymer and (meth)acrylic-modified polycaprolactone compound No. 4 were uniformly mixed at room temperature at a mass ratio of 16.9:83.1. Next, this composition was dispersed in an aqueous solution at 25°C containing 0.27 parts by mass of GOHSENOL EG-05C and 0.53 parts by mass of GOHSENOL EG-18P in 46 parts by mass of pure water, and after uniform emulsification using a colloid mill, 526 parts by mass of pure water was added. After heating in a 1 L flask to 70°C, an aqueous solution of 0.5 g potassium persulfate (manufactured by Sigma-Aldrich) dissolved in 9.5 g water was added dropwise over one minute. The emulsion was stirred at 70°C for three hours and then at 80°C for two hours to perform radical polymerization to prepare a uniform aqueous suspension of silicone rubber particles. The aqueous suspension was then filtered and washed with 200 mL of ethanol and 100 mL of acetone. The residue was dried in an oven at 70°C for 5 hours to obtain silicone elastomer particles No. 4. The average primary and secondary particle diameters of the resulting silicone elastomer particles were 15.24 µm and 592 µm, respectively.

### Comparative Synthesis Example 1: (Meth)acrylic-modified polycaprolactone compound No. C (*without hydrophilic groups)

A four-neck separable flask was filled with 18.81 parts by weight of Placcel 205 (trade name, manufactured by DAICEL CORPORATION, diol type polycaprolactone, mol. wt.: 530), 18.81 parts by weight of chloroform, 8.77 parts by weight of triethyl amine and 0.03 parts by weight of MEHQ (hydroquinonemonomethyl ether, polymerization inhibitor). 6.55 parts by weight of acryloyl chloride was added dropwise while stirring at 200 rpm and aerating with N₂ containing 2% O₂. The reaction was carried out while cooling with a water bath so that the exothermic temperature did not exceed 30°C. After completion of the dropwise addition, the stirring time was extended by 1 hour. Furthermore, the liquid temperature was raised to 50°C, and the mixture was aged for about 2 hours. Next, 28.21 parts by weight of Water-1 was added and stirred well. Thereafter, the mixture was transferred to a separatory funnel, and the bottom phase containing the modified polycaprolactone was removed. Furthermore, 18.81 parts by weight of water-2 was added to make the mixture uniform. The sample was again transferred to a separatory funnel and left overnight for further separation. Chloroform was removed under reduced pressure while bubbling with N₂ containing 2% O₂ to obtain a transparent orange polymer. Based on H-NMR analysis, a peak derived from an acrylic group appeared in the polymer, and it was confirmed that the polymer was polycaprolactone having a terminal modified with an acrylic group ((meth)acryl-modified polycaprolactone compound No. C) having the following structure. 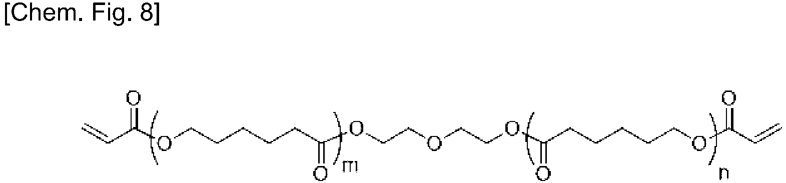 (Where m + n = 3.7.)

### Comparative Example 1: Silicone elastomer particles No. 4 (radical polymerization type)

The methacrylic-modified silicone polymer and (meth)acrylic-modified polycaprolactone compound No. C were uniformly mixed at room temperature at a mass ratio of 30.0:70.0. Next, this composition was dispersed in an aqueous solution at 25°C containing 0.27 parts by mass of GOHSENOL EG-05C and 0.53 parts by mass of GOHSENOL EG-18P in 46 parts by mass of pure water, and after uniform emulsification using a colloid mill, 526 parts by mass of pure water was added. After heating in a 1 L flask to 70°C, an aqueous solution of 0.5 g potassium persulfate (manufactured by Sigma-Aldrich) dissolved in 9.5 g water was added dropwise over one minute. The emulsion was stirred at 70°C for three hours and then at 80°C for two hours to perform radical polymerization to prepare a uniform aqueous suspension of silicone rubber particles. The aqueous suspension was then filtered and washed with 200 mL of ethanol and 100 mL of acetone. The residue was dried in an oven at 70°C for 5 hours to obtain silicone elastomer particles No. 5. The average primary and secondary particle diameters of the resulting silicone elastomer particles were 3.41 µm and 31.1 µm.

The average primary and secondary particle diameters of each particle obtained from the above Examples 1 to 3 and Comparative Example 1 are summarized in Table 1 below.

**[Table 1]**

| | Average primary particle diameter (µm) | Average secondary particle diameter (µm) |
|---|---|---|
| Silicone elastomer particle No. 1 Example 1 | 0.95 | 30.2 |
| Silicone elastomer particle No. 2 Example 2 | 2.84 | 318 |
| Silicone elastomer particle No. 3 Example 3 | 15.24 | 592 |
| Silicone elastomer particle No. 4 Comparative Example 1 | 3.41 | 31.1 |

### Cosmetic Material Formulation Example

The following are formulation examples of cosmetic materials of the present invention that can contain silicone elastomer particles, which is one aspect of the present invention. However, the present invention is not limited thereto.

### Examples 4 to 6 and Comparative Example 2

Panelists compared and evaluated the feel during use of loose powder in which the silicone elastomer particles in the compositions listed in the following Table 3 were used.

**[Table 2]**

| Phase | Component | Product name and supplier | Comparative Example 2 | Examples 4 | Example 5 | Example 6 |
|---|---|---|---|---|---|---|
| A | Dimethicone crosspolymer | Example 1: Silicone elastomer particles No. 1 | | 10 | | |
| | Dimethicone crosspolymer | Example 2: Silicone elastomer particles No. 2 | | | 10 | |
| | Dimethicone crosspolymer | Example 3: Silicone elastomer particles No. 3 | | | | 10 |
| | Phenyl trimethicone | DOWSIL^{™} 556 Cosmetic Fluid | 6 | 6 | 6 | 6 |
| B | Talc | JA-46R available from ASADA MILLING CO., LTD. | 76 | 76 | 76 | 76 |
| | Silylated silica | DOWSIL^{™} VM-2270 Aerogel Fine Particle | 2 | 2 | 2 | 2 |
| | Kaolin | | 1.5 | 1.5 | 1.5 | 1.5 |
| | Titanium oxide | SI Titanium CR-50 available from Miyoshi Kasei, Inc. | 3.92 | 3.92 | 3.92 | 3.92 |
| | Iron oxide yellow | SI-YELLOW-LLXLO available from Miyoshi Kasei, Inc. | 0.46 | 0.46 | 0.46 | 0.46 |
| | Iron oxide red | SA-Bengara Cloisonne available from Miyoshi Kasei, Inc. | 0.09 | 0.09 | 0.09 | 0.09 |
| | Iron oxide black | SA-Black BL-100 available from Miyoshi Kasei, Inc. | 0.02 | 0.02 | 0.02 | 0.02 |
| Evaluation of tactile sensation | | | X | ○ | ○ | ○ |

### Method of preparation

1. Phase A is mixed.
2. Phase B is mixed.
3. Phases A and B are stirred until uniform.

### Evaluation of Tactile Sensation

The 18 panelists evaluated the sliding properties when the samples were applied to the inner forearm.

**[Table 3]**

| Evaluation results | Evaluation Indicators |
|---|---|
| ○ | 12 or more of 18 respondents said the slipperiness was favorable |
| Δ | 7 to 11 of 18 respondents said the slipperiness was favorable |
| X | 6 or fewer of 18 respondents said the slipperiness was favorable |

### Examples 7 to 9 and Comparative Examples 3 and 4

The feel of use of water-in-oil sunscreens was compared and evaluated by a panel of experts. In addition, the SPF and PA values were measured and compared using an SPF analyzer.

**[Table 4]**

| Phase | Component | Product name and supplier | Comparative Example 3 | Comparative Example 4 | Example 7 | Example 8 | Example 9 |
|---|---|---|---|---|---|---|---|
| A | Zinc oxide dispersion (50% zinc oxide diluted with 2cs dimethicone) | | 20 | 20 | 20 | 20 | 20 |
| | Titanium dioxide dispersion (50% titanium dioxide diluted with dimethicone 2cs) | | 36 | 36 | 36 | 36 | 36 |
| | Dimethicone | XIAMETER^{™} PMX-200 2cs | 9.8 | 6.8 | 6.8 | 6.8 | 6.8 |
| | Caprylyl methicone | DOWSIL^{™} FZ-3196 | 10 | 10 | 10 | 10 | 10 |
| | Silicone emulsifiers | DOWSIL^{™} ES-5300 Formulation Aid | 2 | 2 | 2 | 2 | 2 |
| | Dimethicone crosspolymer | DOWSIL^{™} EP-9610 Cosmetic Powder | | 3 | | | |
| | Dimethicone crosspolymer | Example 1: Silicone elastomer particles No. 1 | | | 3 | | |
| | Dimethicone crosspolymer | Example 2: Silicone elastomer particles No. 2 | | | | 3 | |
| | Dimethicone crosspolymer | Example 3: Silicone elastomer particles No. 3 | | | | | 3 |
| B | Sodium citrate | | 0.2 | 0.2 | 0.2 | 0.2 | 0.2 |
| | Sodium chloride | | 0.5 | 0.5 | 0.5 | 0.5 | 0.5 |
| | BG | | 3 | 3 | 3 | 3 | 3 |
| | Water | | 17.8 | 17.8 | 17.8 | 17.8 | 17.8 |
| C | Preservative | Euxyl PE9010 available from Schülke & Mayr | 0.7 | 0.7 | 0.7 | 0.7 | 0.7 |
| Evaluation of tactile sensation | | | X | X | ○ | ○ | ○ to Δ |
| SPF | | | 39 | 22 | 79 | 107 | 35 |
| PA | | | +++ | +++ | ++++ | ++++ | ++++ |

### Method of preparation

Phase A is mixed.
Phase B is mixed.
Phase B is slowly added while stirring Phase A.
Phase C is added to 4 above and then stirred until uniform.

### Evaluation of Tactile Sensation

The 18 panelists evaluated the spreadability and whitening properties when the samples were applied to the inner forearm.

**[Table 5]**

| Evaluation results | Evaluation Indicators |
|---|---|
| ○ | 12 or more of 18 respondents said spreadability is favorable and white residue is less likely to remain |
| Δ | 7 to 11 out of 18 respondents said spreadability is favorable and white residue is less likely to remain |
| X | 6 or less of 18 respondents said spreadability is favorable and white residue is less likely to remain |

### SPF measurement

A sample (2 mg/cm²) was applied to HelioScreen Labs' HELIOPLATE HD6, and SPF and PA values were measured using Labsphere's UV-2000S (the values are the average of 30 measurements taken at 10 locations on each of the three applied samples).

### Examples 10 to 12 and Comparative Examples 5 and 6

The feel of use of water-in-oil foundations were compared and evaluated by a panel of experts.

**[Table 6]**

| Phase | Component | Product name and supplier | Comparative Example 5 | Comparative Example 6 | Example 10 | Example 11 | Example 12 |
|---|---|---|---|---|---|---|---|
| A | Dimethicone | XIAMETER^{™} PMX-200 2cs | 8.55 | 5.55 | 5.55 | 5.55 | 5.55 |
| | Silicone emulsifiers | DOWSIL^{™} ES-5612 Formulation Aid | 4 | 4 | 4 | 4 | 4 |
| | Dimethicone crosspolymer | DOWSIL^{™} EP-9610 Cosmetic Powder | | 3 | | | |
| | Dimethicone crosspolymer | Example 1: Silicone elastomer particles No. 1 | | | 3 | | |
| | Dimethicone crosspolymer | Example 2: Silicone elastomer particles No. 2 | | | | 3 | |
| | Dimethicone crosspolymer | Example 3: Silicone elastomer particles No. 3 | | | | | 3 |
| B | Titanium oxide | SI Titanium CR-50 available from Miyoshi Kasei, Inc. | 10.5 | 10.5 | 10.5 | 10.5 | 10.5 |
| | Iron oxide yellow | SI-YELLOW-LLXLO available from Miyoshi Kasei, Inc. | 1.23 | 1.23 | 1.23 | 1.23 | 1.23 |
| | Iron oxide red | SA-Bengara Cloisonne available from Miyoshi Kasei, Inc. | 0.25 | 0.25 | 0.25 | 0.25 | 0.25 |
| | Iron oxide black | SA-Black BL-100 available from Miyoshi Kasei, Inc. | 0.06 | 0.06 | 0.06 | 0.06 | 0.06 |
| | Dimethicone | XIAMETERTM PMX-200 5cs | 5.45 | 5.45 | 5.45 | 5.45 | 5.45 |
| C | Sodium chloride | | 1 | 1 | 1 | 1 | 1 |
| | Glycerin | | 5 | 5 | 5 | 5 | 5 |
| | Water | | Residual | Residual | Residual | Residual | Residual |
| D | Preservative | Euxyl PE9010 available from Schülke & Mayr | 0.7 | 0.7 | 0.7 | 0.7 | 0.7 |
| Evaluation of tactile sensation | | | X | Poor emulsification | ○ | ○ | ○ to Δ |

### Method of preparation

Phase A is mixed.
Phase B is mixed.
Mix phase A and phase B.
Phase C is slowly added while stirring phases A and B.
Phase D is added to 4 above and then stirred until uniform.

### Evaluation of Tactile Sensation

The 18 panelists evaluated the spreadability and moist feel when the samples were applied to the inner forearm.

**[Table 7]**

| Evaluation results | Evaluation Indicators |
|---|---|
| ○ | 12 or more of 18 persons responded that spreadability was favorable and there was a moist feel after drying |
| Δ | 7 to 11 of 18 persons responded that spreadability was favorable and there was a moist feel after drying |
| X | 6 or fewer of 18 persons responded that spreadability was favorable and there was a moist feel after drying |

### Example 13 and Comparative Examples 7 and 8

The feel of use and blurring effect of oil-in-water type wrinkle concealer creams were compared and evaluated.

**[Table 8]**

| Phase | Component | Product name and supplier | Comparative Example 7 | Comparative Example 8 | Example 13 |
|---|---|---|---|---|---|
| A | Silicone emulsifier premix | ACULYN^{™} Siltouch Rheology Modifier | 2 | 2 | 2 |
| | Cyclopentasiloxane | DOWSIL^{™} SH245 | 20 | 20 | 20 |
| | Dimethicone crosspolymer | DOWSIL^{™} EP-9610 Cosmetic Powder | | 10 | |
| | Dimethicone crosspolymer | Example 3: Silicone elastomer particles No. 3 | | | 10 |
| B | BG | | 3 | 3 | 3 |
| | PEG/PPG-17/16 copolymer | UCON ^{™} Fluid 75H450 | 3 | 3 | 3 |
| | Water | | Residual | Residual | Residual |
| C | Preservative | NEOLONE PH100 | 0.8 | 0.8 | 0.8 |
| Evaluation of tactile sensation | | | X | ○ | ○ |
| Blur effect | | | X | ○ | ○ |

### Method of preparation

Phase A is mixed.
Phase B is mixed.
Phase B is slowly added while stirring Phase A.
Phase C is added to 4 above and then stirred until uniform.

### Evaluation of Tactile Sensation

The 18 panelists evaluated the spreadability and moist feel when the samples were applied to the inner forearm.

**[Table 9]**

| Evaluation results | Evaluation Indicators |
|---|---|
| ○ | 12 or more of 18 persons responded that spreadability was favorable and there was a moist feel after drying |
| Δ | 7 to 11 of 18 persons responded that spreadability was favorable and there was a moist feel after drying |
| X | 6 or fewer of 18 persons responded that spreadability was favorable and there was a moist feel after drying |

### Blur effect

The solution was applied to a microscope slide to a thickness of 10 micrometers, and the blurring effect was determined by the degree to which the text beneath the slide was blurred. The silicone elastomer particles according to the examples of the present invention demonstrated favorable results in all cosmetic compositions.

**[Table 10]**

| Evaluation results | Evaluation Indicators |
|---|---|
| ○ | Text is hard to read |
| Δ | The text appears slightly blurry. |
| X | The letters are clearly visible |

### Enzymatic decomposition test of silicone elastomer particles

Examples 1 to 3 (Silicone elastomer particles No. 1 to 3), Comparative Example 1 (Silicone elastomer particle No. 4), and conventional silicone elastomer products
The prepared silicone elastomer particles and an existing "silicone elastomer product of the applicant" (Dow Toray Industries, product name: EP-9610 Cosmetic Powder; obtained by hydrosilylation of alkenyl modified polysiloxane and organohydrogen polysiloxane, silicone elastomer particles) were each measured out to 0.1 g and placed in an Eppendorf tube. Type XIII lipase derived from bacteria belonging to the genus Pseudomonas was mixed with 0.1 M phosphate buffered saline (pH 7.4) to prepare an 8 U/mL enzyme solution. 1 mL of the obtained enzyme solution was added to each tube to prepare a test sample. The test samples were placed in an oven at 37°C and tested for up to 96 hours with enzyme exchanges every 24 hours. After the specified time, each sample was removed, washed with water, dried overnight and then oven dried in a vacuum oven. The weight of the sample after absolute drying was measured, and the weight reduction rate was defined as the decomposition rate. The enzymatic degradation test (test time-percent degradation) is shown in FIG. 1.

It is presumed that the silicone elastomer particles according to these examples have biodegradability because the weight thereof decreases over time in the presence of an enzyme. In particular, the silicone elastomer particles according to Examples 1 to 3 both showed a high degradation rate of more than 10% after 96 hours, and the silicone elastomer particles according to the examples of the present application are strongly expected to have biodegradability. On the other hand, existing silicone elastomers did not exhibit biodegradability, and the silicone elastomer particles in the comparative example, which lacked hydrophilic groups in the crosslinked moieties, demonstrated a lower decomposition rate compared to Examples 1 to 3.

## Claims

1. Biodegradable silicone elastomer particles having a structure in which at least two silicon atoms in the silicone elastomer particles are crosslinked by a divalent organic group containing a hydrophilic group, and the silicone elastomer particles contain a polyorganosiloxane structure expressed by
-(R₂SiO)ₘ-
(where R represents an unsubstituted alkyl group having 1 to 20 carbon atoms or an alkyl group having 1 to 20 carbon atoms substituted with a halogen atom, an aryl group having 6 to 22 carbon atoms, or a hydroxyl group, and m is a number ranging from 1 to 1000).

2. Biodegradable silicone elastomer particles according to claim 1, wherein a structure crosslinked by a divalent organic group containing a hydrophilic group is derived from at least one type of crosslinking agent selected from:
(A1) a crosslinking agent having a hydrophilic group in the molecule where both terminals of the molecular chain are blocked with a hydrosilylation-reactive silyl group expressed by R^{Alk}R₂SiO (where R^{Alk} is an organic group containing a carbon-carbon double bond, and R is an alkyl group or an aryl group); and
(A2) a crosslinking agent having at least two radical polymerizable functional groups in the molecule and containing a hydrophilic group in the molecule.

3. The biodegradable silicone elastomer particles according to claim 2, wherein
Component (A1) is a crosslinking agent containing (a1-1) a polyether structure in the molecule; and
Component (A2) is one or more type of crosslinking agents selected from:
(a2-1) sorbitan fatty acid esters or polyoxyalkylene sorbitan fatty acid esters having at least two radical polymerizable functional groups in each molecule; and
(a2-2) crosslinking agents containing at least two radical polymerizable functional groups and a polyether structure in each molecule.

4. The biodegradable silicone elastomer particles according to claim 2, wherein
Component (A1) is (a1-1-1) a crosslinking agent containing a polyoxyethylene-polyoxypropylene block copolymer structure in each molecule; and
Component (A2) is one or more type of crosslinking agents selected from:
(a2-1-1) sorbitan lauryl esters, sorbitan stearyl esters, sorbitan oleyl esters, and polyoxyethylene sorbitan oleyl esters having at least two radical polymerizable functional groups in each molecule; and (a2-2-1) crosslinking agents containing at least two radical polymerizable functional groups and a polyoxyethylene-polyoxypropylene block copolymer structure in each molecule.

5. The biodegradable silicone elastomer particles according to claim 1 or claim 2, containing at least:
(A) at least one type of crosslinking agent selected from the following Component (A1) and Component (A2):
Component (A1) is a crosslinking agent having a hydrophilic group in each molecule where both terminal groups of each molecule are blocked with a hydrosilylation reactive silyl group expressed by
R^{Alk}R¹₂SiO
(where R^{Alk} is an organic group containing a carbon-carbon double bond, and R is an alkyl group having 1 to 20 carbon atoms that is unsubstituted or substituted with a halogen atom, an aryl group having 6 to 22 carbon atoms, or a hydroxyl group), and
Component (A2) is a crosslinking agent having at least two radical polymerizable functional groups in the molecule and containing a hydrophilic group in the molecule;
(B) at least one type of reactive organopolysiloxane selected from the following Component (b1) and Component (b2):
(b1) an organohydrogenpolysiloxane having at least three silicon atom-bonded hydrogen atoms in a molecule, and
(b2) an organopolysiloxane having at least three organic groups containing at least one type of (meth)acryloxy group selected from organic groups containing a methacryloxy group and organic groups containing an acryloxy group in each molecule; and
(C) one or more type of curing agent selected from (c1) a hydrosilylation reaction catalyst, and (c2) a radical polymerization initiator;
obtained by performing in water a crosslinking reaction of crosslinkable silicone emulsified particles obtained by emulsifying in water a crosslinkable silicone composition crosslinkable by one or more types of reactions selected from radical polymerization reactions and hydrosilylation reactions with a silicon atom-bonded hydrogen atom.

6. The biodegradable silicone elastomer particles according to claim 1, made by cross-link reacting in water crosslinkable emulsified particles made by emulsifying in water a crosslinkable reactive silicone composition that can be crosslinked by a radical polymerization reaction, containing:
(A1) a crosslinking agent having a hydrophilic group in each molecule where both terminal groups of each molecule are blocked with a hydrosilylation reactive silyl group expressed by R^{Alk}R¹₂SiO (where R^{Alk} is an organic group containing a carbon-carbon double bond, and R is an alkyl group having 1 to 20 carbon atoms that is unsubstituted or substituted with a halogen atom, an aryl group having 6 to 22 carbon atoms, or a hydroxyl group);
(b1) an organohydrogenpolysiloxane having at least three silicon atom-bonded hydrogen atoms in a molecule; and
(c2) a hydrosilylation reaction catalyst.

7. The biodegradable silicone elastomer particles according to claim 1, made by cross-link reacting in water crosslinkable emulsified particles made by emulsifying in water a crosslinkable reactive silicone composition that can be crosslinked by a radical polymerization reaction, containing:
(A2) a crosslinking agent having at least two radical polymerizable functional groups in the molecule and containing a hydrophilic group in the molecule;
(b2) an organopolysiloxane having at least three organic groups containing a (meth)acryloxy group of at least one type selected from organic groups containing a methacryloxy group and organic groups containing an acryloxy group in each molecule; and
(c2) a radical polymerization initiator.

8. The biodegradable silicone elastomer particles according to any one of claims 1 to 7, wherein the average primary particle diameter as measured by a laser diffraction scattering method is 0.5 to 20 µm.

9. The biodegradable silicone elastomer particles according to any one of claims 1 to 7, wherein the radical polymerizable polycarbonate modified silicone compound used in forming the particles is cured in a sheet form, and the JIS-A hardness that is measured is in a range of 10 to 80.

10. The biodegradable silicone elastomer particles according to any one of claims 1 to 7, further comprising a structure in which some or all of the surface thereof is covered with one or more types selected from organopolysiloxane resins, silica, and other silicone elastomer particles.

11. The biodegradable silicone elastomer particles according to any one of claims 1 to 7, wherein the particles have a mesoporous structure.

12. The biodegradable silicone elastomer particles according to any one of claims 1 to 7, wherein the particles contain an oil that is liquid at 40°C.

13. The biodegradable silicone elastomer particles according to any one of claims 1 to 12, wherein the divalent organic groups containing hydrophilic groups in the particle are active in biodegradation reactions, and in a biodegradable environment, the crosslinked structures formed between silicon atoms in the particle are at least partially cleaved, and the primary particles are disintegrated with generation of non-crosslinked polyorganosiloxanes.

14. A cosmetic raw material, comprising the biodegradable silicone elastomer particles according to any one of Claims 1 to 13.

15. A cosmetic composition, comprising the biodegradable silicone elastomer particles according to any one of Claims 1 to 13.

16. An organic resin additive, comprising the biodegradable silicone elastomer particles according to any one of claims 1 to 13.

17. An organic resin, comprising the biodegradable silicone elastomer particles according to any one of claims 1 to 13.
